# EUROPÄISCHE PATENTSCHRIFT

(11) **EP 2 619 193 B1**
(45) Veröffentlichungstag und Bekanntmachung des Hinweises auf die Patenterteilung: **12.11.2014**
(21) Anmeldenummer: 11749868.3
(22) Anmeldetag: 05.09.2011
(51) Int. Cl.: C07D 307/08

(54) **VERFAHREN ZUR GEWINNUNG VON TETRAHYDROFURAN**
PROCESS FOR THE PRODUCTION OF TETRAHYDROFURAN
PROCÉDÉ DE PRODUCTION DE TÉTRAHYDROFURANE

(30) Priorität: 24.09.2010 EP 10179309
(43) Veröffentlichungstag der Anmeldung: 31.07.2013
(73) Patentinhaber: BASF SE, 67056 Ludwigshafen (DE)
(72) Erfinder: GUIXA GUARDIA, Maria, 40547 Düsseldorf (DE); PINKOS, Rolf, 67098 Bad Dürkheim (DE); POLLMER, Nadja, 67281 Bissersheim (DE); WEIßKER, Wolf-Steffen, 67245 Lambsheim (DE); VANDENMERSCH, Hugues, Jardine's Lookout Hong Kong (CN)
(86) Internationale Anmeldenummer: PCT/EP2011/065314
(87) Internationale Veröffentlichungsnummer: WO 2012/038242

(56) Entgegenhaltungen:
- WO-A1-88/00937
- GB-A- 1 402 507

## Beschreibung

Die Erfindung geht aus von einem Verfahren zur Gewinnung von Tetrahydrofuran aus einem Tetrahydrofuran, Alkanol und Schwersieder enthaltenden Stoffstrom, bei dem in einem ersten Destillationsschritt ein Tetrahydrofuran und Alkanol als Azeotrop enthaltender Stoffstrom abgetrennt wird und in einem weiteren Destillationsschritt aus dem Azeotrop Tetrahydrofuran als Produkt gewonnen wird.

Die Herstellung von Tetrahydrofuran erfolgt im Allgemeinen gemeinsam mit der Herstellung von Butan-1,4-diol und γ-Butyrolacton. Zur Herstellung wird im Allgemeinen in einer ersten Stufe Maleinsäureanhydrid, das z.B. durch Oxidation aus Butan bzw. Benzol gewonnen wird, mit einem Alkanol zu Monoalkylmaleat verestert. Die Veresterung des Maleinsäureanhydrids mit dem Alkanol erfolgt üblicherweise unkatalysiert. In einer zweiten Veresterung wird das Monoalkylmaleat mit Alkanol zu Dialkylmaleat umgesetzt. Die Umsetzung des Monoalkylmaleats zu Dialkylmaleat erfolgt üblicherweise katalysiert und wird in einer Reaktionskolonne durchgeführt. Neben einer zweistufigen Umsetzung ist auch eine einstufige Umsetzung von Maleinsäureanhydrid zu Dialkylmaleat möglich.

Das Dialkylmaleat wird in Abhängigkeit vom eingesetzten Katalysatorsystem in einem weiteren Schritt zu einem Gemisch aus Butan-1,4-diol, Tetrahydrofuran und γ-Butyrolacton, einem Gemisch aus Tetrahydrofuran und γ-Butyrolacton oder zu Tetrahydrofuran hydriert. Ein entsprechendes Verfahren zur Herstellung von Butan-1,4-diol, γ-Butyrolacton und Tetrahydrofuran ist zum Beispiel in WO 99/48852 beschrieben. Auch aus EP-A 0 255 399, WO 97/43242 und US 4,751,334 sind entsprechende Verfahren zur Herstellung von Butan-1,4-diol, γ-Butyrolacton und Tetrahydrofuran bekannt.

Um die einzelnen Reaktionsprodukte zu gewinnen, wird das Butan-1,4-diol, γ-Butyrolacton und Tetrahydrofuran enthaltende Gemisch in einer ersten Kolonne destilliert. In dieser wird über Kopf ein Alkanol und Tetrahydrofuran als Azeotrop enthaltendes Gemisch abgezogen. Zur Gewinnung des Tetrahydrofurans wird dieses Gemisch einer zweiten Destillationskolonne zugeführt, in der Tetrahydrofuran als Schwersieder abgezogen wird und über Kopf ein Tetrahydrofuran und Alkanol enthaltendes Gemisch in die erste Destillationskolonne zurückgeführt wird. Alkanol, Butan-1,4-diol und γ-Butyrolacton werden am Sumpf der ersten Destillationskolonne entnommen und einer weiteren Verarbeitung zugeführt. Die Aufarbeitung des Butan-1,4-diol, γ-Butyrolacton und Tetrahydrofuran enthaltenen Produktgemischs ist zum Beispiel in DE-C 29 27 931 oder auch in US 5,310,954 beschrieben.

Das gesamte Verfahren zur Herstellung von Butan-1,4-diol, bei dem auch Tetrahydrofuran entsteht sowie der Aufarbeitung des Produktgemischs ist zum Beispiel aus WO-A 91/01960 bekannt.

Nachteil der beschriebenen Verfahren ist jeweils, dass ein relativ großer Anteil an Tetrahydrofuran zusammen mit dem Alkanol über Kopf in der zweiten Destillationskolonne abgetrennt wird, so dass ein großer Anteil an Produkt wieder zurück in die erste Destillationskolonne geführt wird.

Daher ist es Aufgabe der vorliegenden Erfindung, ein Verfahren zur Gewinnung von Tetrahydrofuran aus einem Tetrahydrofuran und Alkanol als Azeotrop enthaltenden Stoffstrom bereitzustellen, in dem der zurückzuführende Methanol und Tetrahydrofuran enthaltende Strom minimiert wird und sich so die Kapazität für Tetrahydrofuran der Destillationssequenz steigern lässt.

Gelöst wird die Aufgabe durch ein Verfahren zur Gewinnung von Tetrahydrofuran aus einem Tetrahydrofuran, Alkanol und Schwersieder enthaltenden Stoffstrom, welches folgende Schritte umfasst:
(a) Abtrennen eines Tetrahydrofuran und Alkanol als Azeotrop enthaltenden Stoffstroms in einer ersten Destillationsstufe,
(b) Zufuhr mindestens eines Teils des Tetrahydrofuran und Alkanol als Azeotrop enthaltenden Stoffstroms in einen Reaktor zur Veresterung von Maleinsäureanhydrid, wobei ein Tetrahydrofuran, gegebenenfalls Alkanol, gegebenenfalls nicht umgesetztes Maleinsäureanhydrid und Monoalkylmaleat enthaltender Stoffstrom erhalten wird,
(c) Trennen des Monoalkylmaleat, Tetrahydrofuran und gegebenenfalls Alkanol enthaltenden Stoffstroms in einen Monoalkylmaleat enthaltenden Stoffstrom und einen Tetrahydrofuran und gegebenenfalls Alkanol enthaltenden Stoffstrom in einer zweiten Destillationsstufe,
(d) Zufuhr des Tetrahydrofuran und gegebenenfalls Alkanol enthaltenen Stoffstroms aus der zweiten Destillationsstufe sowie des nicht dem Reaktor zugeführten Teils des Tetrahydrofuran und Alkanol als Azeotrop enthaltenden Stoffstroms aus Schritt (a) in eine dritte Destillationsstufe, in der ein im Wesentlichen Tetrahydrofuran enthaltender Produktstrom und ein Tetrahydrofuran und Alkanol enthaltender Stoffstrom erhalten werden,
(e) Zurückführen des Tetrahydrofuran und Alkanol enthaltenen Stoffstroms aus der dritten Destillationsstufe in die erste Destillationsstufe oder in den Reaktor in Schritt (b).

Durch die Zufuhr des mindestens einen Teils des Tetrahydrofuran und Alkanol als Azeotrop enthaltenen Stoffstroms in einen Reaktor zur Veresterung von Maleinsäureanhydrid, in dem ein Tetrahydrofuran, Alkanol und Monoalkylmaleat enthaltender Stoffstrom erhalten wird und Trennen dieses Stoffstroms in einen Monoalkylmaleat enthaltenden Stoffstrom und einen Tetrahydrofuran und Alkanol enthaltenden Stoffstrom wird zumindest ein Teil des in dem Tetrahydrofuran und Alkanol als Azeotrop enthaltenden Stoffstrom enthaltenen Alkanols bei der Reaktion verbraucht, so dass ein größerer Anteil an Tetrahydrofuran zusammen mit dem gegebenenfalls verbleibenden Alkanol am dritten Destillationsschritt anfällt. Der Anteil an Tetrahydrofuran, das als Produkt gewonnen wird, wird auf diese Weise erhöht. In dem bei der Reaktion in Schritt (b) anfallenden Stoffstrom ist kein Alkanol enthalten, wenn das Alkanol in der Reaktion vollständig umgesetzt wird. Üblicherweise erfolgt jedoch kein Vollumsatz, so dass Alkanol in dem Stoffstrom enthalten ist.

Im Rahmen der vorliegenden Erfindung bedeutet "im Wesentlichen Tetrahydrofuran enthaltend", dass im Stoffstrom mindestens 98Gew.-%, bevorzugt mindestens 98,5 Gew.-% Tetrahydrofuran enthalten sind.

Der Tetrahydrofuran, Alkanol und Schwersieder enthaltende Stoffstrom entstammt üblicherweise einer Hydrierung, in der Dialkylmaleat hydriert wird. Neben Tetrahydrofuran und Alkanol kann der Stoffstrom im Allgemeinen Butan-1,4-diol und/oder γ-Butyrolacton enthalten. In Abhängigkeit vom eingesetzten Katalysatorsystem enthält der Stoffstrom Tetrahydrofuran, Butan-1,4-diol und γ-Butyrolacton; Tetrahydrofuran und γ-Butyrolacton; Tetrahydrofuran und Butan-1,4-diol oder nur Tetrahydrofuran. Diese werden als Schwersieder in der ersten Destillationsstufe abgetrennt. Neben den Wertstoffen Tetrahydrofuran, γ-Butyrolacton und Butan-1,4-diol ist auch das entstehende Alkanol sowie im Allgemeinen Wasser im Stoffstrom enthalten. Das Wasser und das Alkanol werden ebenfalls als Schwersieder im ersten Destillationsschritt abgetrennt. Im Allgemeinen enthält der Tetrahydrofuran, Alkanol und Schwersieder enthaltende Stoffstrom 2 bis 30 Gew.-% Tetrahydrofuran, 0 bis 20 Gew.-% γ-Butyrolacton, 20 bis 50 Gew.-% 1,4-Butandiol, 30 bis 50 Gew.-% Alkanol, 0 bis 8 Gew.-% Wasser, 0 bis 2 Gew.-% Butanol und 0 bis 5 Gew.-% Dimethylsuccinat. Bevorzugt enthält der Tetrahydrofuran, Alkanol und Schwersieder enthaltende Stoffstrom 5 bis 15 Gew.-% Tetrahydrofuran, 5 bis 15 Gew.-% γ-Butyrolacton, 30 bis 45 Gew.-% 1,4-Butandiol, 35 bis 45 Gew.-% Alkanol, 1 bis 5 Gew.-% Wasser, 0,2 bis 1,5 Gew.-% Butanol und 0 bis 1,5 Gew.-% Dimethylsuccinat.

Bei dem Verfahren zur Herstellung des Tetrahydrofurans wird im Allgemeinen in einem ersten Veresterungsreaktor Maleinsäureanhydrid mit Alkanol zu Monoalkylmaleat in einer Gleichgewichtsreaktion umgesetzt. Das Monoalkylmaleat wird in einem zweiten Veresterungsreaktor mit Alkanol zu einem Dialkylmaleat umgesetzt und anschließend wird das Dialkylmaleat zu Tetrahydrofuran sowie gegebenenfalls Butan-1,4-diol und/oder γ-Butyrolacton hydriert.

Das Alkanol, das zur Herstellung des Tetrahydrofurans aus Maleinsäureanhydrid eingesetzt wird, ist im Allgemeinen ein C₁-C₄-Alkanol. Geeignete C₁-C₄-Alkanole umfassen insbesondere Methanol, Ethanol, n-Propanol, iso-Propanol und Butanol. Bevorzugt als Alkanole eingesetzt werden Methanol und Ethanol, insbesondere Methanol.

Die Umsetzung des Maleinsäureanhydrids mit Alkanol zu Monoalkylmaleat im ersten Veresterungsreaktor erfolgt üblicherweise nicht katalytisch. In dieser Reaktion kann bereits eine Umsetzung des Monoalkylmaleats zum entsprechenden Dialkylmaleat auftreten.

Der erste Veresterungsreaktor, in dem eine nicht-katalytische Umsetzung des Maleinsäureanhydrids stattfindet, wird üblicherweise bei einer Temperatur im Bereich von 65 °C bis 260 °C und einem Druck im Bereich von 1 bis 50 bar betrieben. An den ersten Veresterungsreaktor schließt sich üblicherweise ein zweiter Veresterungsreaktor an, in dem eine katalytische Veresterung durchgeführt wird. Die zweite Veresterungsstufe kann mehrere Rührkesselreaktoren umfassen, wie zum Beispiel in US 4,795,824 beschrieben ist. Vorzugsweise umfasst die katalytische Veresterungsstufe jedoch eine Reaktivkolonne, wie sie beispielsweise in WO-A 90/03127 beschrieben ist. In diesem Fall kann die erste Veresterungsstufe einen Rührkessel als Reaktor oder eine Reaktivkolonne mit einem oder mehreren Böden umfassen, wobei kein Veresterungskatalysator enthalten ist. Die Reaktivkolonne wird am Boden mit Alkanoldampf beschickt, während die Maleinsäureanhydrid-Lösung im Gegenstrom durch die Reaktivkolonne geleitet wird.

Wenn die zweite Veresterungsstufe, die katalytisch betrieben wird, eine Reaktivkolonne enthält, wird die das Monoalkylmaleat enthaltende Lösung dem obersten Boden der Reaktivkolonne zugeführt, während ein Überschuss an Alkanoldampf am Sumpf der Reaktivkolonne zugeführt wird.

In der katalytisch betriebenen zweiten Veresterungsstufe enthält jeder Boden der Reaktivkolonne eine Charge Veresterungskatalysator.

Neben einer Bodenkolonne als Reaktivkolonne ist es alternativ auch möglich, zum Beispiel eine Packungskolonne als Reaktivkolonne einzusetzen, wobei die Packung den Katalysator enthält.

Wenn eine Bodenkolonne eingesetzt wird, so können beliebige Böden, beispielsweise Siebböden oder Glockenböden verwendet werden.

Typische Reaktionsbedingungen, mit denen die Reaktivkolonne betrieben wird, umfassen eine Temperatur und einen Druck, unter denen das eingesetzte Alkanol destilliert. Diese Temperatur- und Druckbedingungen variieren in Abhängigkeit vom ausgewählten Alkanol. Üblicherweise liegen die Temperaturen im Bereich von 65 bis 135 °C und der Druck im Bereich von 1 bis 3 bar. Als Veresterungskatalysator kann zum Beispiel ein Ionenaustauschharz, das unter der Bezeichnung Amberlyst^{™} von Rohm & Haas vertrieben wird, eingesetzt werden.

Säuren, die im Maleinsäureanhydrid vorhanden sind, beispielsweise Essigsäure oder Acrylsäure, werden zusammen mit Maleinsäure oder Fumarsäure, die in der der ersten Veresterungsstufe zugeführten Lösung anwesend ist, zu dem entsprechenden C₁-C₄-Alkylester oder -diester umgesetzt.

Am Kopf der Reaktivkolonne wird ein Stoffstrom abgezogen, der C₁-C₄-Alkanoldampf und Wasserdampf enthält. Weiterhin können Spuren von untergeordneten Nebenprodukten wie Dialkylether, Spuren des Dialkylmaleats und des Alkylacrylats enthalten sein. Um das Dialkylmaleat zurückzuführen, können oberhalb des obersten Veresterungsbodens zusätzliche Böden vorgesehen werden, die als Waschsäule wirken, in der das Dialkylmaleat ausgewaschen wird. Im Allgemeinen enthält der am Kopf der Reaktivkolonne abgezogene Stoffstrom 30 bis 90 Gew.-% Alkanol, 10 bis 50 Gew.-% Wasser und Spuren untergeordneter Nebenkomponenten.

In diesem Fall tritt ein Dampfstrom, der im Wesentlichen frei von Dialkylmaleat ist, am Kopf der Reaktivkolonne aus.

Am Sumpf der Reaktivkolonne wird ein flüssiger Strom gewonnen, der eine Lösung des Dialkylmaleats enthält. Im Allgemeinen enthält der am Sumpf der Reaktivkolonne gewonnene Strom mehr als 80 Gew.-% des Dialkylmaleats, weniger als 20 % Alkanol und Spuren von untergeordneten Nebenkomponenten.

Neben der vorstehend beschriebenen zweistufigen Veresterung kann die Veresterung des Maleinsäureanhydrid enthaltenden Stroms auch einstufig erfolgen. Diese Einstufige Vorgehensweise ist zum Beispiel in EP-A 0 062 874 beschrieben. In einer geeigneten Ausführungsform führt man zum Beispiel den Maleinsäureanhydrid enthaltenden Strom und das Alkanol zusammen mit einem Katalysator, der im Allgemeinen sauer ist (z. B. Schwefelsäure oder ein saurer Ionentauscher) am Kopf einer geeigneten als Reaktionskolonne dienenden Bodenkolonne zu. Bei der Veresterung entstehendes Wasser wird in Form von wässrigem Alkohol am Kopf der Kolonne entnommen, wobei der entnommene wässrige Alkohol in einer separaten Anlage entwässert wird. Das Alkanol wird gegebenenfalls in die Kolonne zurückgeführt. Am Sumpf der Reaktionskolonne wird ein Dialkylmaleat enthaltender Strom abgezogen.

Der Dialkylmaleat enthaltende Stoffstrom, der in der zweiten Veresterungsstufe oder im einstufigen Verfahren gewonnen wird, wird einer Hydrierung zugeführt. Die Hydrierung wird vorteilhafterweise in der Dampfphase unter Verwendung eines heterogenen Ester-Hydrierkatalysators durchgeführt. Als Ester-Hydrierkatalysatoren eignen sich zum Beispiel reduzierte Kupferkatalysatoren mit Promotor, zum Beispiel reduzierte Kupferchromit-Katalysatoren.

Die Katalysatorteilchen haben vorzugsweise eine Teilchengröße im Bereich von 0,5 bis 5 mm. Die Teilchen können beliebige geeignete Form haben, zum Beispiel Kugeln, Pellets, Ringe oder Sättel. Bei Benutzung eines Katalysator-Festbetts kann der Reaktor ein Rohrbündelreaktor sein, der im Wesentlichen isotherm arbeiten kann. Bevorzugt wird jedoch ein adiabater Reaktor.

Die Hydrierung wird im Allgemeinen bei einer erhöhten Temperatur im Bereich von 150 bis 240 °C und bei einem Druck im Bereich von 5 bis 100 bar, vorzugsweise von 50 bis 70 bar durchgeführt. Eine solche Hydrierung ist zum Beispiel in WO-A 88/00937; WO-A 91/01960, US 4,751,334, WO-A 03/006446 oder WO-A 05/058855 beschrieben.

Durch die Hydrierung wird ein Produktgemisch gewonnen, das neben dem eingesetzten C₁-C₄-Alkanol Tetrahydrofuran sowie gegebenenfalls Butan-1,4-diol und/oder γ-Butyrolacton enthält. Dieser Strom enthält zusätzlich u. a. n-Butanol, Dimethylsuccinat und Wasser. Dieses Produktgemisch wird als Tetrahydrofuran, Alkanol und Schwersieder enthaltender Stoffstrom dem Verfahren zur Gewinnung von Tetrahydrofuran in Schritt (a) zugeführt.

In einer ersten Ausführungsform der Erfindung ist der Reaktor zur Veresterung von Maleinsäureanhydrid, dem ein Teil des Tetrahydrofuran und Alkanol als Azeotrop enthaltenden Stoffstroms zugeführt wird, der Reaktor der ersten Veresterungsstufe des Verfahrens zur Herstellung von Tetrahydrofuran, dem der Tetrahydrofuran, Alkanol und Schwersieder enthaltende Stoffstrom entstammt.

Überraschenderweise hat sich gezeigt, dass das Tetrahydrofuran, das zusammen mit dem Alkanol der ersten Veresterungsstufe zugegeben wird, keine negativen Einflüsse auf die erste Veresterungsstufe hat.

Nach Durchführung der ersten Veresterungsstufe wird der in der Veresterungsstufe erhaltene Monoalkylmaleat enthaltende Stoffstrom, der auch das nicht umgesetzte Tetrahydrofuran enthält, der Destillation in Schritt (c) zugeführt. Hier wird das Monoalkylmaleat als Schwersieder aus dem Tetrahydrofuran und einem Rest Alkanol enthaltenden Stoffstrom abgetrennt. Das Monoalkylmaleat wird der zweiten Veresterungsstufe zugeführt und der über Kopf abgetrennte Leichtsiederstrom, der Tetrahydrofuran und Alkanol enthält, wird der dritten Destillationsstufe in Schritt (d) zur Abtrennung des Tetrahydrofurans zugeführt.

In einer alternativen Ausführungsform sind der Reaktor zur Veresterung von Maleinsäureanhydrid in Schritt (b) und der Reaktor der ersten Veresterungsstufe zwei voneinander getrennte Reaktoren. Der Reaktor, der zur Veresterung von Maleinsäureanhydrid in Schritt (b) eingesetzt wird, kann dabei im Aufbau dem Reaktor der ersten Veresterungsstufe entsprechen. Weiterhin sind auch die Verfahrensbedingungen, unter denen das Maleinsäureanhydrid im Reaktor verestert wird, die gleichen wie die der ersten Veresterungsstufe des Hauptverfahrens. Hauptverfahren ist dabei das Verfahren zur Herstellung von Tetrahydrofuran, Butan-1,4-diol und γ-Butyrolacton, aus dem der Tetrahydrofuran, Alkanol und Schwersieder enthaltende Stoffstrom stammt, der im ersten Destillationsschritt (a) destilliert wird.

Wenn zur Veresterung des Maleinsäureanhydrids in Schritt (b) ein separater Reaktor eingesetzt wird, so wird diesem Reaktor Maleinsäureanhydrid in einer Menge zugegeben, dass das Molverhältnis von Säureanhydrid zu Alkohol zwischen 1: 10 und 10 : 1, bevorzugt zwischen 5 : 1 und 1 : 5 und besonders bevorzugt zwischen 3:1 und 1:3 liegt. Der Reaktor kann kontinuierlich oder batchweise betrieben werden. Geeignete Reaktoren sind zum Beispiel Rohrreaktoren oder Rührkesselreaktoren. Die Reaktion der ersten Veresterungsstufe, in der das Alkanol mit Maleinsäureanhydrid zum Monoalkylmaleat umgesetzt wird, liegt vorzugsweise im Bereich von 50 bis 150 °C, insbesondere 60 bis 130 °C bei einem Druck, bei dem der Alkohol in der Flüssigphase verbleibt, beispielsweise im Bereich von 1 bis 20 bar. Die Verweilzeit wird vorzugsweise im Bereich zwischen 5 bis 300 Minuten, bevorzugt 5-120 min, beispielsweise 60 Minuten, gewählt. Die Verweilzeit wird dabei vorzugsweise so eingestellt, dass der Methanol-Umsatz zwischen 30 und 100%, insbesondere zwischen 40 und 85% liegt. Neben dem Monoalkylmaleat, Tetrahydrofuran und Alkanol kann das in der ersten Veresterungsstufe erhaltene Gemisch zusätzlich auch Dialkylmaleat, nicht abreagiertes Maleinsäureanhydrid und Wasser enthalten.

Der den Reaktor zur Veresterung verlassende, Tetrahydrofuran, gegebenenfalls Alkanol und Monoalkylmaleat enthaltende Stoffstrom wird der zweiten Destillationsstufe (c) zugeführt. In dieser werden Tetrahydrofuran und Alkanol vom Monoalkylmaleat getrennt. Die zweite Destillationsstufe wird so betrieben, dass der in der dritten Destillationsstufe (d) abgetrennte im Wesentlichen Tetrahydrofuran enthaltende Stoffstrom die vorgegebene Reinheit aufweist. Das heißt, dass der der dritten Destillationsstufe (d) zugeführte Stoffstrom keine Schwersieder wie Monoalkylmaleat, Dialkylmaleat und Maleinsäureanhydrid mehr enthält.

Der in der zweiten Destillationsstufe (c) erhaltene Monoalkylmaleat enthaltende Strom kann neben dem Monoalkylmaleat auch Maleinsäureanhydrid, Tetrahydrofuran und Alkanol enthalten. Dieser Monoalkylmaleat enthaltende Strom wird weiter verarbeitet. Hierzu ist es insbesondere bevorzugt, das Monoalkylmaleat der ersten oder zweiten Veresterungsstufe des Hauptverfahrens zuzuführen, in der dieses zu Dialkylmaleat umgesetzt wird. Im Allgemeinen enthält der Monoalkylmaleat enthaltende Stoffstrom 50 bis 95 Gew.-% Monoalkylmaleat, 1 bis 50 Gew.-% Maleinsäureanhydrid, 0 bis 5 Gew.-% Dialkylmaleat, 0 bis 5 Gew.-% Tetrahydrofuran, 0 bis 3 Gew.-% Wasser sowie Spuren von untergeordneten Nebenkomponenten.

Der erste Destillationsschritt (a), in dem Tetrahydrofuran und Alkanol als Azeotrop aus dem Tetrahydrofuran, Alkanol und Schwersieder enthaltenden Stoffstrom abgetrennt werden, wird im Allgemeinen in einer ersten Destillationskolonne durchgeführt. Die erste Destillationskolonne wird bei einem Druck im Bereich von 0,1 bis 5 bar, bevorzugt bei nicht mehr als 2 bar, beispielsweise bei 1,1 bar, betrieben. Als Destillationskolonne wird eine Kolonne üblicher Bauart eingesetzt. Geeignete Kolonnen sind zum Beispiel Glockenbodenkolonnen, Ventilbodenkolonnen oder Füllkörperkolonnen. Auch eine Kolonne, die eine geordnete Packung enthält, kann eingesetzt werden.

Die Destillationskolonne weist vorzugsweise 20 bis 60 theoretische Trennstufen und Rücklaufverhältnisse zwischen 0,5 und 5 auf. Am Sumpf der Destillationskolonne werden die Schwersieder, insbesondere Butan-1,4-diol und γ-Butyrolacton sowie Wasser und Alkanol entnommen. Am Kopf der Kolonne wird der Tetrahydrofuran und Alkanol als Azeotrop enthaltende Stoffstrom entnommen.

Die Zusammensetzung des Tetrahydrofuran und Alkanol als Azeotrop enthaltenden Stoffstroms ist abhängig vom eingesetzten Alkanol und dem Druck, bei dem der Stoffstrom entnommen wird. Wenn Alkanol enthalten ist, liegen Tetrahydrofuran und Alkanol üblicherweise als Azeotrop vor. So bilden zum Beispiel Tetrahydrofuran und Methanol ein Azeotrop bei einem Druck von 1,2 bar und einem Verhältnis von Tetrahydrofuran zu Methanol von 0,67 zu 0,33. Bei einem Druck von 1,4 bar ist das Verhältnis von Tetrahydrofuran zu Methanol 0,65 zu 0,35. Tetrahydrofuran und Ethanol bilden bei einem Druck von 1,1 bar ein Azeotrop mit 92,5 Gew.-% Tetrahydrofuran und 7,5 Gew.-% Ethanol und bei einem Druck von 1,5 bar ein Azeotrop mit 88,9 Gew.-% Tetrahydrofuran und 11,1 Gew.-% Ethanol.

Die zweite Destillationsstufe, in der verbleibendes Alkanol und Tetrahydrofuran vom Monoalkylmaleat getrennt werden, kann ebenfalls in jeder beliebigen, geeigneten Destillationsvorrichtung durchgeführt werden. Auch hier ist insbesondere eine Destillationskolonne geeignet. Alternativ können Tetrahydrofuran und Alkanol auch vom Monoalkylmaleat zum Beispiel in einem geeigneten Verdampfer, beispielsweise einem Fallfilmverdampfer oder Dünnschichtverdampfer abgetrennt werden. Bevorzugt ist jedoch eine Destillationskolonne. Die Destillationskolonne ist auch hier vorzugsweise eine Kolonne üblicher Bauart, beispielsweise eine Glockenbodenkolonne, eine Ventilbodenkolonne, eine Füllkörperkolonne oder eine Kolonne mit geordneter Packung. Die Kolonne weist vorzugsweise 1 bis 30 theoretische Trennstufen und ein Rücklaufverhältnis im Bereich von 0,1 bis 5 auf.

Der Druck, bei dem die zweite Destillationsstufe betrieben wird, liegt vorzugsweise im Bereich von 0,1 bis 1,2 bar und ist üblicherweise kleiner als der Druck im ersten Destillationsschritt.

Der in der zweiten Destillationsstufe aus dem Monoalkylmaleat, Tetrahydrofuran und gegebenenfalls Alkanol enthaltenden Stoffstrom abgetrennte, gegebenenfalls Alkanol und Tetrahydrofuran enthaltende Stoffstrom wird zusammen mit dem nicht dem Reaktor zugeführten Tetrahydrofuran und Alkanol als Azeotrop enthaltenden Stoffstrom einer dritten Destillationsstufe zugeführt. Die dritte Destillationsstufe (Schritt (d)) wird vorzugsweise ebenfalls in einer Destillationskolonne durchgeführt. Auch hier kann eine Kolonne üblicher Bauart, beispielsweise eine Glockenbodenkolonne, eine Ventilbodenkolonne, eine Füllkörperkolonne oder eine Kolonne mit geordneter Packung eingesetzt werden. Die Destillationskolonne der dritten Destillationsstufe weist vorzugsweise 15 bis 50 theoretische Stufen auf und wird bei einem höheren Druck als die Kolonne der ersten Destillationsstufe, beispielsweise bei einem Druck im Bereich zwischen 3 und 25 bar, bevorzugt zwischen 6 und 10 bar, betrieben. Am Kopf der Destillationskolonne der dritten Destillationsstufe fällt ein Tetrahydrofuran und Alkanol enthaltender Stoffstrom an, der zurück in die erste Destillationsstufe (a) geleitet wird. Am Sumpf der Destillationskolonne der dritten Destillationsstufe wird der im Wesentlichen Tetrahydrofuran enthaltende Produktstrom entnommen.

Der Teil des Tetrahydrofuran und Alkanol als Azeotrop enthaltenden Stoffstroms, der dem Reaktor zur Veresterung von Maleinsäureanhydrid in Schritt (b) zugeführt wird, liegt vorzugsweise im Bereich von 20 bis 100 Gew.-%. Bevorzugt liegt der Anteil im Bereich von 30 bis 80 Gew.-%, beispielsweise bei 50 Gew.-%.

Ausführungsbeispiele der Erfindung sind in den Figuren dargestellt und werden in der nachfolgenden Beschreibung näher erläutert.

Es zeigen:
- Figur 1: eine schematische Darstellung des erfindungsgemäßen Verfahrens,
- Figur 2: ein Verfahrensfließbild des erfindungsgemäßen Verfahrens.

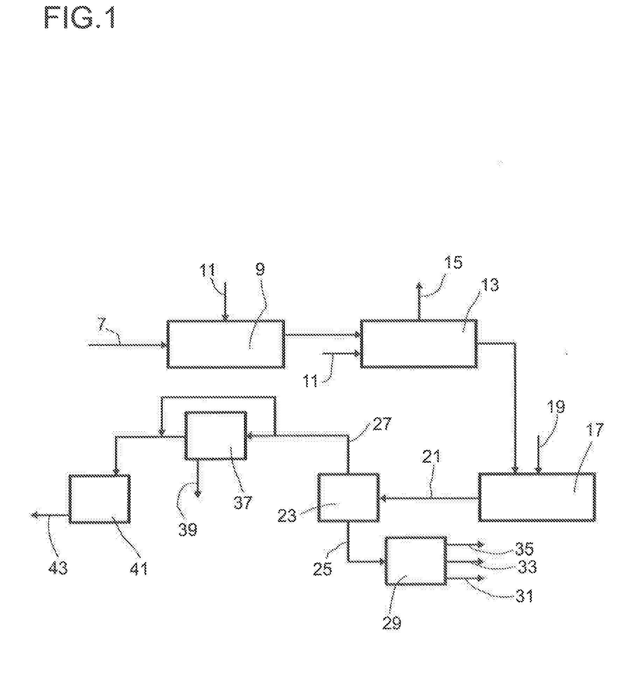

In Figur 1 ist das erfindungsgemäße Verfahren schematisch dargestellt.

Über eine Leitung 7 wird Maleinsäureanhydrid einer ersten Veresterungsstufe 9 zugeführt. Neben dem Maleinsäureanhydrid 7 wird der ersten Veresterungsstufe 9 auch ein C₁-C₄-Alkanol 11 zugeführt. In der ersten Veresterungsstufe 9 wird der Maleinsäureanhydrid mit dem C₁-C₄-Alkanol zu einem Monoalkylmaleat umgesetzt.

Das zugeführte C₁-C₄-Alkanol ist vorzugsweise Methanol oder Ethanol, so dass das in der ersten Veresterungsstufe 9 hergestellte Monoalkylmaleat Monomethylmaleat oder Monoethylmaleat ist.

Das in der ersten Veresterungsstufe 9 hergestellte Monoalkylmaleat wird zusammen mit dem nicht umgesetzten Maleinsäureanhydrid und nicht umgesetztem C₁-C₄-Alkanol einer zweiten Veresterungsstufe 13 zugeführt. Neben dem Monoalkylmaleat enthaltenden Strom aus der ersten Veresterungsstufe 9 wird der zweiten Veresterungsstufe 13 weiteres C₁-C₄-Alkanol 11 zugeführt. In der zweiten Veresterungsstufe 13 wird das Monoalkylmaleat mit dem zusätzlichen C₁-C₄-Alkanol zu Dialkylmaleat umgesetzt. Das C₁-C₄-Alkanol, das der zweiten Veresterungsstufe 13 zugeführt wird, ist dabei üblicherweise das gleiche C₁-C₄-Alkanol wie das, das der ersten Veresterungsstufe 9 zugeführt wird. Wenn der ersten Veresterungsstufe 9 somit Methanol zugeführt wird, wird der zweiten Veresterungsstufe 13 auch Methanol zugeführt.

Bei der Veresterung in der zweiten Veresterungsstufe 13 entstandenes Wasser sowie nicht umgesetztes C₁-C₄-Alkanol wird über einen Ablauf 15 entnommen.

Das in der zweiten Veresterungsstufe 13 erzeugte Dialkylmaleat wird einer Hydrierstufe 17 zugeführt. Zur Hydrierung wird der Hydrierstufe 17 weiterhin Wasserstoff 19 zugegeben. In der Hydrierstufe 17 wird das Dimethylmaleat mit dem Wasserstoff zu Tetrahydrofuran sowie gegebenenfalls Butan-1,4-diol und/oder γ-Butyrolacton unter gleichzeitiger Bildung von Alkanol und Wasser umgesetzt, wie zum Beispiel in WO 88/00937, WO 91/01960, WO 03/006446 oder WO 05/058855 beschrieben. Das in der Hydrierung entstehende Alkanol ist dabei das gleiche Alkanol wie das, das der ersten Veresterungsstufe 9 und der zweiten Veresterungsstufe 13 zugegeben wurde.

Der in der Hydrierstufe hergestellte, Butan-1,4-diol, Tetrahydrofuran, γ-Butyrolacton, Alkanol und Wasser enthaltende Rohproduktstrom 21 wird einer ersten Destillationsstufe 23 zugeführt. In der ersten Destillationsstufe 23 wird der Rohproduktstrom 21, der Tetrahydrofuran und Alkanol sowie Schwersieder enthaltende Stoffstrom, in einen Schwersieder enthaltenden Sumpfstrom 25 und einen Tetrahydrofuran und Alkanol als Azeotrop enthaltenden Kopfstrom 27 getrennt. Der Schwersieder enthaltende Sumpfstrom 25, der Butan-1,4-diol, γ-Butyrolacton, Alkanol und Wasser enthält, wird einer Schwersiederaufarbeitung 29 zugeführt, in der dieser Strom in γ-Butyrolacton 31, Butan-1,4-diol 33 und Alkanol und Wasser 35 aufgeteilt wird.

Der der ersten Destillationsstufe 23 entnommene Tetrahydrofuran und Alkanol als Azeotrop enthaltende Kopfstrom 27 wird vollständig oder teilweise einer Veresterungsstufe 37 zugeführt, der weiterhin Maleinsäureanhydrid zugegeben wird. In der Veresterungsstufe 37 wird der Maleinsäureanhydrid mit dem im Kopfstrom 27 enthaltenen Alkanol zu Monoalkylmaleat 39 umgesetzt. Das Monoalkylmaleat 39 wird vorzugsweise der ersten Veresterungsstufe 11 oder der zweiten Veresterungsstufe 13 zugegeben. Zur Abtrennung des Monoalkylmaleats 39 umfasst die Veresterungsstufe 37 eine Destillationsstufe, in der das Monoalkylmaleat von dem nicht reagierten Alkanol und Tetrahydrofuran getrennt wird.

Das in der Veresterungsstufe 37 nicht umgesetzte Alkanol sowie das Tetrahydrofuran werden einer dritten Destillationsstufe 41 zugeführt. Neben dem aus der Veresterungsstufe 37 abgeführten Tetrahydrofuran und Alkanol wird auch der Teil des Tetrahydrofuran und Alkanol als Azeotrop enthaltenden Stoffstroms 27, der der Veresterungsstufe 37 nicht zugeführt wurde, der dritten Destillationsstufe 41 zugeführt. In der dritten Destillationsstufe 41 wird der Alkanol und Tetrahydrofuran enthaltende Stoffstrom in einen im Wesentlichen Tetrahydrofuran enthaltenen Produktstrom 43 und einen Tetrahydrofuran und Alkanol enthaltenden zweiten Strom aufgetrennt, wobei der Tetrahydrofuran und Alkanol enthaltende Strom zurück in die erste Destillationsstufe 23 geleitet wird.

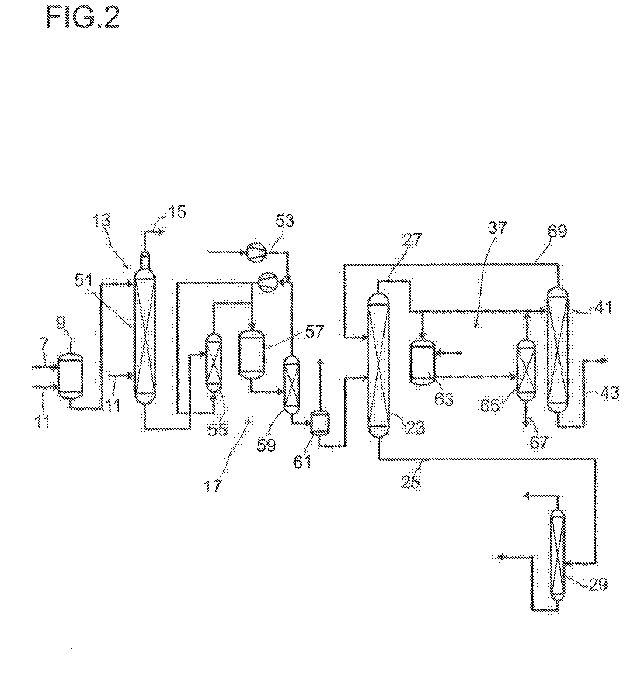

In Figur 2 ist das erfindungsgemäße Verfahren beispielhaft als Verfahrensfließbild dargestellt.

Der ersten Veresterungsstufe 9 wird über einen ersten Zulauf 7 Maleinsäureanhydrid und über einen zweiten Zulauf das C₁-C₄-Alkanol 11 zugeführt. Die Zufuhr kann über getrennte Zuläufe, wie in Figur 2 dargestellt, oder auch gemeinsam über einen Zulauf erfolgen, wobei in diesem Fall die Zuleitung für das C₁-C₄-Alkanol in die Leitung für das Maleinsäureanhydrid mündet. Als Reaktor für die erste Veresterungsstufe 9 wird ein Reaktor mit dem Fachmann bekannter Bauart eingesetzt, beispielsweise ein Rohrreaktor oder ein Rührkessel. Eine Kaskade von Reaktoren ist auch möglich.

Der in der ersten Veresterungsstufe 9 erzeugte Monoalkylmaleat, C₁-C₄-Alkanol sowie nicht abreagiertes Maleinsäureanhydrid enthaltende Stoffstrom wird der zweiten Veresterungsstufe 13 zugeführt. Die zweite Veresterungsstufe 13 wird zum Beispiel in einer Reaktivkolonne 51 durchgeführt. Die Zufuhr des Monoalkylmaleat enthaltenden Stoffstroms erfolgt vorzugsweise im oberen Bereich der Reaktivkolonne 51 und die Zufuhr des für die Veresterung des Monoalkylmaleats zu Dialkylmaleat benötigten C₁-C₄-Alkanols 11 erfolgt vorzugsweise im unteren Bereich der Reaktivkolonne 51. Das Monoalkylmaleat und das C₁-C₄-Alkanol werden so im Gegenstrom in der Reaktivkolonne 51 geführt.

In der Reaktivkolonne 51 wird gleichzeitig der entstehende Produktstrom destilliert, und über Kopf wird nicht abreagiertes C₁-C₄-Alkanol sowie bei der Veresterung entstehendes Wasser abgezogen. Am Sumpf der Reaktivkolonne 51 wird das in der Reaktivkolonne 51 erzeugte Dialkylmaleat abgezogen. Dieses wird der Hydrierstufe 17 zugeführt. Über eine Zuleitung 53 wird der Hydrierstufe 17 Wasserstoff zugeführt.

Das Dialkylmaleat wird zunächst einem Verdampfer 55 zugeführt, der auch von Wasserstoff durchströmt wird. Im Verdampfer 55 wird das Dialkylmaleat verdampft und mit dem Wasserstoff gemischt. Das verdampfte Dialkylmaleat wird zusammen mit dem Wasserstoff einem Hydrierreaktor 57 zugeführt. Im Hydrierreaktor 57 wird das Dialkylmaleat zu Butan-1,4-diol, γ-Butyrolacton und Tetrahydrofuran umgesetzt. Der Produktstrom sowie Nebenprodukte und nicht abreagierte Edukte werden in einem Kondensator 59 auskondensiert und der nicht umgesetzte Wasserstoff wird abgetrennt. In einem Entspannungsbehälter 61 wird der Produktstrom entspannt und dabei freiwerdendes zuvor gelöstes Gas wird abgetrennt.

Der Tetrahydrofuran, Alkanol und Schwersieder enthaltende Rohproduktstrom wird anschließend der ersten Destillationsstufe 23 zugeführt. Als Schwersieder sind in dem Rohproduktstrom Butan-1,4-diol, γ-Butyrolacton, Wasser und nicht umgesetztes Alkanol enthalten. Diese werden am Sumpf der ersten Destillationsstufe 23, die üblicherweise als Destillationskolonne ausgebildet ist, abgezogen und der Schwersiederaufarbeitung 29 zugeführt. In der Schwersiederaufarbeitung 29 werden über Kopf Alkanol und Wasser und am Sumpf γ-Butyrolacton und Butan-1,4-diol gewonnen. Der Sumpfstrom wird dann weiter in γ-Butyrolacton und Butan-1,4-diol getrennt.

Am Kopf der Destillationskolonne der ersten Destillationsstufe 23 wird ein Tetrahydrofuran und Alkanol als Azeotrop enthaltender Kopfstrom 27 abgezogen. Ein Teil dieses Tetrahydrofuran und Alkanol als Azeotrop enthaltenden Kopfstroms 27 oder der gesamte Tetrahydrofuran und Alkanol als Azeotrop enthaltende Kopfstrom 27 wird der Veresterungsstufe 37 zugeführt. Die erste Veresterungsstufe 37 umfasst dabei einen Veresterungsreaktor 63 und eine zweite Destillationsstufe 65. Dem Veresterungsreaktor 63 wird neben dem Alkanol und Tetrahydrofuran als Azeotrop enthaltenden Stoffstrom Maleinsäureanhydrid zugeführt. Das Maleinsäureanhydrid reagiert mit dem Alkanol des Tetrahydrofuran und Alkanol als Azeotrop enthaltenden Stoffstroms zu Monoalkylmaleat. Der gesamte Produktstrom wird der zweiten Destillationsstufe 65 zugeführt, in der Monoalkylmaleat als Sumpfstrom 67 abgezogen wird und nicht umgesetztes Alkanol und Tetrahydrofuran über Kopf. Der über Kopf der zweiten Destillationsstufe 65 entnommene Tetrahydrofuran und Alkanol enthaltende Strom wird zusammen mit dem nicht dem Veresterungsreaktor 63 zugeführten Teil des Tetrahydrofuran und Alkanol als Azeotrop enthaltenden Kopfstrom 27 der dritten Destillationsstufe 41 zugegeben. Die dritte Destillationsstufe 41 ist dabei vorzugsweise ebenfalls als Destillationskolonne ausgebildet. Am Kopf der dritten Destillationsstufe 41 fällt ein Tetrahydrofuran und Alkanol enthaltender Kopfstrom 69 an, der in die erste Destillationsstufe 63 zurückgeleitet wird. Am Sumpf der dritten Destillationsstufe wird der im Wesentlichen Tetrahydrofuran enthaltende Produktstrom 43 gewonnen.

Neben der in Figur 2 dargestellten Ausführungsform, bei der der Veresterungsreaktor 63 ein eigenständiger Reaktor ist, ist es auch möglich, den Reaktor der ersten Veresterungsstufe 9 zu nutzen. In diesem Fall wird der Teil des Tetrahydrofuran und Alkanol als Azeotrop enthaltenden Kopfstroms 27 nicht einem Reaktor 63 zugeführt, sondern in den Reaktor der ersten Veresterungsstufe 9 geleitet. Wenn der Reaktor der ersten Veresterungsstufe 9 genutzt wird, wird an den Reaktor anschließend zunächst die Abtrennung von Tetrahydrofuran und Alkanol in der zweiten Destillationsstufe 65 durchgeführt, bevor das Monoalkylmaleat, das in der ersten Veresterungsstufe 9 erzeugt wurde, in die zweite Veresterungsstufe 13 geleitet wird.

Bei Verwendung eines separaten Reaktors 63, wie es in Figur 2 dargestellt ist, ist es ebenfalls bevorzugt, das in der zweiten Destillationsstufe 65 gewonnene Monoalkylmaleat in die erste Veresterungsstufe 11 oder in die zweite Veresterungsstufe 13 zu leiten.

### Bezugszeichenliste

- 1: erster Reaktor
- 3: n-Butan
- 5: Luft
- 7: Leitung für Maleinsäureanhydrid
- 9: erste Veresterungsstufe
- 11: C₁-C₄-Alkanol
- 13: zweite Veresterungsstufe
- 15: Ablauf für H₂O-Alkanol
- 17: Hydrierstufe
- 19: Wasserstoff
- 21: Rohprodukt
- 23: erste Destillationsstufe
- 25: Schwersieder enthaltender Sumpfstrom
- 27: Tetrahydrofuran und Alkanol enthaltender Kopfstrom
- 29: Schwersiederaufarbeitung
- 31: γ-Butyrolacton
- 33: Butan-1,4-diol
- 35: Alkanol und Wasser
- 37: Veresterungsstufe
- 39: Monoalkylmaleat
- 41: dritte Destillationsstufe
- 43: Tetrahydrofuran enthaltender Produktstrom
- 51: Reaktivkolonne
- 53: H₂-Zuleitung
- 55: Verdampfer
- 57: Hydrierreaktor
- 59: Kondensator
- 61: Gasabscheider
- 63: Veresterungsreaktor
- 65: zweite Destillationsstufe
- 67: Sumpfstrom
- 69: Tetrahydrofuran und Alkanol enthaltender Kopfstrom

## Patentansprüche

1. Verfahren zur Gewinnung von Tetrahydrofuran aus einem Tetrahydrofuran, Alkanol und Schwersieder enthaltenden Stoffstrom, folgende Schritte umfassend:
(a) Abtrennen eines Tetrahydrofuran und Alkanol als Azeotrop enthaltenden Stoffstroms in einer ersten Destillationsstufe,
(b) Zufuhr mindestens eines Teils des Tetrahydrofuran und Alkanol als Azeotrop enthaltenden Stoffstroms in einen Reaktor zur Veresterung von Maleinsäureanhydrid, wobei ein Tetrahydrofuran, gegebenenfalls Alkanol, gegebenenfalls nicht umgesetztes Maleinsäureanhydrid und Monoalkylmaleat enthaltender Stoffstrom erhalten wird,
(c) Trennen des Monoalkylmaleat, Tetrahydrofuran und gegebenenfalls Alkanol enthaltenden Stoffstroms in einen Monoalkylmaleat enthaltenden Stoffstrom und einen Tetrahydrofuran und gegebenenfalls Alkanol enthaltenden Stoffstrom in einer zweiten Destillationsstufe,
(d) Zufuhr des Tetrahydrofuran und gegebenenfalls Alkanol enthaltenen Stoffstroms aus der zweiten Destillationsstufe sowie des nicht dem Reaktor zugeführten Teils des Tetrahydrofuran und Alkanol als Azeotrop enthaltenden Stoffstroms aus Schritt (a) in eine dritte Destillationsstufe, in der ein im Wesentlichen Tetrahydrofuran enthaltender Produktstrom und ein Tetrahydrofuran und Alkanol enthaltender Stoffstrom erhalten werden,
(e) Zurückführen des Tetrahydrofuran und Alkanol enthaltenen Stoffstroms aus der dritten Destillationsstufe in die erste Destillationsstufe oder in den Reaktor in Schritt (b).

2. Verfahren gemäß Anspruch 1, **dadurch gekennzeichnet, dass** der Tetrahydrofuran, Alkanol und Schwersieder enthaltende Stoffstrom einem Verfahren zur Herstellung von Tetrahydrofuran entstammt, in dem Maleinsäureanhydrid in einer ersten Veresterungsstufe mit Alkanol zu Monoalkylmaleat umgesetzt wird, das Monoalkylmaleat in einer zweiten Veresterungsstufe mit Alkanol zu einem Dialkylmaleat umgesetzt wird und anschließend das Dialkylmaleat zu Tetrahydrofuran, Butan-1,4-diol und γ-Butyrolacton hydriert wird.

3. Verfahren gemäß Anspruch 1 oder 2, **dadurch gekennzeichnet, dass** die im Tetrahydrofuran, Alkanol und Schwersieder enthaltenden Stoffstrom enthaltenen Schwersieder Butan-1,4-diol, γ-Butyrolacton und Wasser umfassen.

4. Verfahren gemäß Anspruch 2 oder 3, **dadurch gekennzeichnet, dass** der Reaktor zur Veresterung von Maleinsäureanhydrid in Schritt (b) der Reaktor der ersten Veresterungsstufe ist.

5. Verfahren gemäß einem der Ansprüche 1 bis 3, **dadurch gekennzeichnet, dass** der Reaktor zur Veresterung von Maleinsäureanhydrid in Schritt (b) und der Reaktor der ersten Veresterungsstufe zwei voneinander getrennte Reaktoren sind.

6. Verfahren gemäß Anspruch 5, **dadurch gekennzeichnet, dass** das im Reaktor zur Veresterung von Maleinsäureanhydrid in Schritt (b) hergestellte Monoalkylmaleat der ersten oder zweiten Veresterungsstufe zugeführt wird.

7. Verfahren gemäß einem der Ansprüche 1 bis 6, **dadurch gekennzeichnet, dass** dem Reaktor zur Veresterung von Maleinsäureanhydrid in Schritt (b) 20 bis 100 Gew.-% des Tetrahydrofuran und Alkanol als Azeotrop enthaltenden Stoffstroms zugeführt werden.

8. Verfahren gemäß einem der Ansprüche 1 bis 7, **dadurch gekennzeichnet, dass** das Alkanol Methanol oder Ethanol ist.

9. Verfahren gemäß einem der Ansprüche 1 bis 8, **dadurch gekennzeichnet, dass** der Reaktor zur Veresterung von Maleinsäureanhydrid in Schritt (b) so betrieben wird, dass der Umsatz an Alkanol in dem Reaktor im Bereich von 30 bis 100% liegt.

## Claims

1. A process for isolating tetrahydrofuran from a stream comprising tetrahydrofuran, alkanol and high boilers, which comprises the following steps:
(a) separating off a stream comprising tetrahydrofuran and alkanol as azeotrope in a first distillation stage,
(b) feeding at least part of the stream comprising tetrahydrofuran and alkanol as azeotrope into a reactor for esterifying maleic anhydride, giving a stream comprising tetrahydrofuran, optionally alkanol, optionally unreacted maleic anhydride and monoalkyl maleate,
(c) separating the stream comprising monoalkyl maleate, tetrahydrofuran and optionally alkanol into a stream comprising monoalkyl maleate and a stream comprising tetrahydrofuran and optionally alkanol in a second distillation stage,
(d) feeding the stream comprising tetrahydrofuran and optionally alkanol from the second distillation stage and the part of the stream comprising tetrahydrofuran and alkanol as azeotrope from step (a) which is not fed to the reactor into a third distillation stage in which a product stream comprising essentially tetrahydrofuran and a stream comprising tetrahydrofuran and alkanol are obtained,
(e) recirculating the stream comprising tetrahydrofuran and alkanol from the third distillation stage to the first distillation stage or to the reactor in step (b).

2. The process according to claim 1, wherein the stream comprising tetrahydrofuran, alkanol and high boilers originates from a process for preparing tetrahydrofuran, in which maleic anhydride is reacted with alkanol to form monoalkyl maleate in a first esterification stage, the monoalkyl maleate is reacted with alkanol to form a dialkyl maleate in a second esterification stage and the dialkyl maleate is subsequently hydrogenated to tetrahydrofuran, 1,4-butanediol and γ-butyrolactone.

3. The process according to claim 1 or 2, wherein the high boilers comprised in the stream comprising tetrahydrofuran, alkanol and high boilers comprise 1,4-butanediol, γ-butyrolactone and water.

4. The process according to claim 2 or 3, wherein the reactor for esterifying maleic anhydride in step (b) is the reactor of the first esterification stage.

5. The process according to any of claims 1 to 3, wherein the reactor for esterifying maleic anhydride in step (b) and the reactor of the first esterification stage are two separate reactors.

6. The process according to claim 5, wherein the monoalkyl maleate produced in the reactor for esterifying maleic anhydride in step (b) is fed to the first or second esterification stage.

7. The process according to any of claims 1 to 6, wherein from 20 to 100% by weight of the stream comprising tetrahydrofuran and alkanol as azeotrope is fed to the reactor for esterifying maleic anhydride in step (b).

8. The process according to any of claims 1 to 7, wherein the alkanol is methanol or ethanol.

9. The process according to any of claims 1 to 8, wherein the reactor for esterifying maleic anhydride in step (b) is operated so that the conversion of alkanol in the reactor is in the range from 30 to 100%.

## Revendications

1. Procédé pour l'obtention de tétrahydrofurane à partir d'un courant de substances contenant du tétrahydrofurane, un alcanol et des composants à haut point d'ébullition, comprenant les étapes suivantes :
(a) dans un premier étage de distillation, fractionnement d'un courant de substances contenant du tétrahydrofurane et un alcanol sous forme d'azéotrope
(b) envoi d'au moins une partie du courant de substances contenant du tétrahydrofurane et un alcanol sous forme d'azéotrope dans un réacteur destiné à l'estérification d'anhydride maléique, pour l'obtention d'un courant de substances contenant du tétrahydrofurane, éventuellement un alcanol, éventuellement de l'anhydride maléique n'ayant pas réagi et un maléate de monoalkyle,
(c) dans un deuxième étage de distillation, fractionnement du courant de substances contenant un maléate de monoalkyle, du tétrahydrofurane et éventuellement un alcanol, en un courant de substances contenant un maléate de monoalkyle et un courant de substances contenant du tétrahydrofurane et éventuellement un alcanol,
(d) envoi du courant de substances contenant du tétrahydrofurane et éventuellement un alcanol, provenant du deuxième étage de distillation, ainsi que de la partie du courant de substances provenant de l'étape (a), contenant du tétrahydrofurane et un alcanol sous forme d'azéotrope, non envoyée au réacteur, dans un troisième étage de distillation, dans lequel on obtient un courant de produit contenant essentiellement du tétrahydrofurane et un courant de substances contenant du tétrahydrofurane et un alcanol,
(e) recyclage du courant de substances contenant du tétrahydrofurane et un alcanol, provenant du troisième étage de distillation, dans le premier étage de distillation ou dans le réacteur dans l'étape (b).

2. Procédé selon la revendication 1, **caractérisé en ce que** le courant de substances contenant du tétrahydrofurane, un alcanol et des composants à haut point d'ébullition provient d'un processus destiné à la production de tétrahydrofurane, dans lequel on fait réagir dans un premier stade d'estérification de l'anhydride maléique avec un alcanol pour aboutir à un maléate de monoalkyle, on fait réagir dans un deuxième stade d'estérification le maléate de monoalkyle avec un alcanol pour aboutir à un maléate de dialkyle et ensuite on soumet le maléate de dialkyle à une hydrogénation conduisant à du tétrahydrofurane, du butane-1,4-diol et de la γ-butyrolactone.

3. Procédé selon la revendication 1 ou 2, **caractérisé en ce que** les composants à haut point d'ébullition contenus dans le courant de substances contenant du tétrahydrofurane, un alcanol et des composants à haut point d'ébullition comprennent du butane-1,4-diol, de la γ-butyrolactone et de l'eau.

4. Procédé selon la revendication 2 ou 3, **caractérisé en ce que** le réacteur destiné à l'estérification d'anhydride maléique dans l'étape (b) est le réacteur du premier stade d'estérification.

5. Procédé selon l'une quelconque des revendications 1 à 3, **caractérisé en ce que** le réacteur destiné à l'estérification d'anhydride maléique dans l'étape (b) et le réacteur du premier stade d'estérification sont deux réacteurs séparés l'un de l'autre.

6. Procédé selon la revendication 5, **caractérisé en ce que** le maléate de monoalkyle produit dans le réacteur destiné à l'estérification d'anhydride maléique dans l'étape (b) est envoyé au premier ou au deuxième stade d'estérification.

7. Procédé selon l'une quelconque des revendications 1 à 6, **caractérisé en ce que** dans l'étape (b) on envoie au réacteur destiné à l'estérification d'anhydride maléique 20 à 100 % en poids du courant de substances contenant du tétrahydrofurane et un alcanol sous forme d'azéotrope.

8. Procédé selon l'une quelconque des revendications 1 à 7, **caractérisé en ce que** l'alcanol est le méthanol ou l'éthanol.

9. Procédé selon l'une quelconque des revendications 1 à 8, **caractérisé en ce que** dans l'étape (b) on fait fonctionner le réacteur destiné à l'estérification d'anhydride maléique de manière que le degré de conversion de l'alcanol dans le réacteur se situe dans la plage de 30 à 100 %.
